Europäisches Patentamt

European Patent Office

Office européen des brevets

(19)

(11) Veröffentlichungsnummer : **0 042 119**
**B1**

(12) **EUROPÄISCHE PATENTSCHRIFT**

(45) Veröffentlichungstag der Patentschrift :
**21.03.84**

(21) Anmeldenummer : **81104343.9**

(22) Anmeldetag : **05.06.81**

(51) Int. Cl.³ : **C 07 C 85/08, C 07 C 87/36,**
**C 07 D 211/58, B 01 J 31/08,**
**B 01 J 27/24, B 01 J 23/74**

(54) **Verfahren zur Herstellung primärer Mono- und Diamine aus Oxoverbindungen.**

(30) Priorität : **12.06.80 DE 3021955**

(43) Veröffentlichungstag der Anmeldung :
**23.12.81 Patentblatt 81/51**

(45) Bekanntmachung des Hinweises auf die Patenterteilung : **21.03.84 Patentblatt 84/12**

(84) Benannte Vertragsstaaten :
**BE CH DE FR GB IT LI LU NL SE**

(56) Entgegenhaltungen :
**CH-A- 428 751**
**DE-A- 1 543 377**
**DE-A- 1 620 429**
**DE-A- 2 412 750**
**DE-B- 1 229 078**
**GB-A- 1 130 212**
**US-A- 1 762 742**
**US-A- 3 187 047**
**US-A- 4 041 080**

(73) Patentinhaber : **CHEMISCHE WERKE HÜLS AG**
**Postfach 1320**
**D-4370 Marl 1 (DE)**

(72) Erfinder : **Disteldorf, Josef, Dr.**
**Am Sengenhoff 2a**
**D-4690 Herne 1 (DE)**
Erfinder : **Hübel, Werner, Dr.**
**Birnenbruchstrasse 34**
**D-4690 Herne 1 (DE)**
Erfinder : **Broschinski, Lothar, Dr.**
**Lüenbrink 18**
**D-4760 Werl (DE)**

(74) Vertreter : **Steil, Hanna, Dipl.-Chem.**
**RSP PATENTE - PB 15 Postfach 1320**
**D-4370 Marl 1 (DE)**

EP 0 042 119 B1

# Verfahren zur Herstellung primärer Mono- und Diamine aus Oxoverbindungen

## Verfahrensbeschreibung

Die Erfindung betrifft die Verbesserung des Verfahrens zur Herstellung von primären Mono- und Diaminen durch reduktive Aminierung von Oxoverbindungen, die gegebenenfalls zusätzlich noch andere, zur Reduktion befähigte Gruppen oder Funktionen wie C=C-Doppelbindungen, Nitril-(und Nitro-) Gruppen enthalten können, durch eine Vorbehandlung mit Ammoniak in Gegenwart von mit Ammonium-Ionen beladenen Kontakten.

Als Oxoverbindungen werden wie üblich Carbonylgruppen enthaltende Verbindungen bezeichnet, insbesondere Aldehyde und Ketone.

Bereits bekannt ist, daß sich gesättigte und ungesättigte Aldehyde und Ketone mit Ammoniak und Wasserstoff unter erhöhtem Druck und bei erhöhter Temperatur an geeigneten Katalysatoren, z. B. mit Kobalt und/oder Nickel und/oder Eisen als Hauptkomponenten, in primäre Amine überführen lassen.

Zwar gelingt die reduktive Aminierung von einfach strukturierten niedermolekularen Carbonylverbindungen verhältnismäßig befriedigend, doch bei höher molekularen, kompliziert strukturierten oder polyfunktionellen Verbindungen wird häufig eine verhältnismäßig geringe Raumzeit-Ausbeute beobachtet. Auch die Katalysatorstandzeit ist dann häufig unbefriedigend. Diese beiden Kenngrößen werden durch mehr oder weniger starke Nebenproduktbildung beeinflußt, besonders wenn die Ausbildung cyclischer Strukturen begünstigt ist. Solche Nebenprodukte wirken nicht allein ausbeutemindernd, sondern sie beeinträchtigen wegen der Schwierigkeiten bei der Aufarbeitung, bzw. der Reindarstellung der gewünschten Amine oftmals erheblich die Verfahrensausbeute überhaupt. Ausbeuten unter 80 %, teilsweise sogar unter 50 % werden z. B. beobachtet bei der Herstellung von Benzylamin aus Benzaldehyd, Furfurylamin aus Furfurol, Pentandiamine aus den entsprechenden Cyanoaldehyden, 3,3,5-Trimethylcyclohexylamin (TMCA) aus Isophoron (= 3,5,5-Trimethyl-2-cyclohexen-1-on) oder 2,2,6,6-Tetramethyl-4-aminopiperidin (=Triacetondiamin = TAD) aus 2,2,6,6-Tetramethyl-4-piperidon (= Triacetonamin = TAA) und vor allem für die Herstellung von 3-(Aminomethyl) 3,5,5-Trimethylcyclohexylamin (= Isophorondiamin = IPD) aus 3-Cyan-3,5,5-trimethylcyclohexanon (= Isophoronnitril = IPN). Bei der zuletzt genannten Reaktion entsteht z. B. nicht nur das Nebenprodukt 3-(Aminomethyl) 3,5,5-trimethylcyclohexanol (= Isophoronaminoalkohol = IPAA), sondern es bilden sich durch Abspaltung von HCN, durch Cyclisierung, Konkurenzreaktionen und Weiterreaktion von Zwischenprodukten eine große Zahl von Nebenprodukten. Davon ist ein im Gaschromatographen durch den sogenannten Peak 7 charakterisiertes Verbindungsgemisch besonders schwierig abtrennbar. Dieses Verbindungsgemisch verursacht bei der Reindarstellung von spezifikationsgerechtem IPD, wie es beispielsweise für die Polyamiderzeugung gefordert wird, pro 0,1 % Anteil des Peak-7-Gemisches einen Destillationsverlust von ca. 1 % IPD. Ähnliche unerwünschte Nebenprodukte treten auch bei der reduktiven Aminierung anderer Oxoverbindungen auf.

Es entstand daher die Aufgabe, eine Arbeitsweise zu finden, in der die gewünschten Amine in deutlich höherer Ausbeute bei gleichzeitiger wirtschaftlicher Verfahrensweise im technischen Maßstab erhalten werden.

Überraschenderweise konnte dieses Ziel dadurch erreicht werden, daß man die betreffende Oxoverbindung zunächst mit Ammoniak in Gegenwart eines Iminbildungskatakysators in eine Schiffsche Base überführt und anschließend das so gewonnene Zwischenprodukt der üblichen reduktiven Aminierung unterwirft. Die Überführung einer Oxoverbindung in die Schiff'sche Base gelang befiedigend rasch mit Hilfe von mit Ammoniumionen beladenen anorganischen oder organischen Ionenaustauschern. Die katalytisch aktive Ammoniumform der Ionenaustauscher kann entweder durch vorheriges Beladen der Ionenaustauscher mit Ammoniak oder Aminen, oder während der erfindungsgemäßen Vorreaktion durch Einwirkung von Ammoniak oder Aminen erhalten werden.

Die erfindungsgemäße Vorreaktion zwischen der Oxoverbindung und Ammoniak erfolgt in Gegenwart eines der oben genannten Iminbildungskatalysatoren bei Temperaturen von 10 bis 120 °C, vorzugsweise bei 15 bis 70 °C, unter Eigendruck oder erhöhtem Druck.

Für die Umsetzung von IPN mit Ammoniak beispielsweise liegt für die Bildungsreaktion der Schiff'schen Base (SB) ein Gleichgewicht gemäß der folgenden Formel vor:

$$IPN + NH_3 \rightleftharpoons SB + H_2O$$

Die Gleichgewichtskonstante K ergibt sich dann zu

$$K = \frac{[IPN]\ [NH_3]}{[SB]\ [H_2O]}$$

Daraus geht hervor, daß die Reaktion einer Oxoverbindung zur Schiff'schen Base sowohl durch einen $NH_3$-Überschuß als auch durch die Entfernung von Wasser begünstigt werden kann.

Will man das Reaktionswasser ausschleusen, um das Gleichgewicht zu beeinflussen, dann ist es zweckmäßig, den Ammoniaküberschuß niedrig zu halten.

Empfehlenswert ist in diesem Fall ein Molverhältnis IPN : $NH_3$ wie 1 : 1,3 bis 3. Diese Arbeitsweise ist günstig für die Phasentrennung, höhere $NH_3$-Überschüsse wirken dagegen als Lösungsvermittler und erschweren dadurch die Trennung von wässriger und organischer Phase.

Zur Erleichterung der Phasentrennung kann auch noch Wasser und/oder ein inertes organisches Lösungsmittel zugegeben werden. Durch die Abtrennung der ammoniakalisch-wässrigen Phase wird nicht nur das Gleichgewicht günstig beeinflußt, sondern auch ein höhere Kontaktbelastung bei der Hydrierung ermöglicht.

Verfahrenstechnisch einfacher ist es, von vornherein mit einem hohen $NH_3$-Überschuß zu arbeiten. Ein höherer $NH_3$-Überschuß ist ohnehin bei der reduktiven Aminierung üblich, um dadurch Nebenreaktionen, wie beispielsweise die Bildung sekundärer Aminfunktionen zu unterdrücken. Für die erfindungsgemäße Vorreaktion wird durch den $NH_3$-Überschuß die Bildung der Schiff'schen Base begünstigt. Außerdem bleibt dabei die Reaktionsmischung homogen, und der Gesamtprozeß kann ohne Zwischenoperationen ablaufen.

Die Umsetzung der Oxoverbindung mit Ammoniak kann unter Eigendruck erfolgen. Diese Verfahrensvariante empfieht sich besonders dann, wenn eine Phasentrennung erfolgen soll.

Man kann jedoch die Vorreaktion auch bei höherem Druck, beispielsweise bei 300 bar durchführen. Diese Verfahrensweise ist bei Einsatz eines $NH_3$-Überschusses zu bevorzugen, und besonders dann, wenn die Vorreaktion und reduktive Aminierung in einem Zuge durchgeführt werden soll. Nach der Vorreaktion erfolgt die reduktive Aminierung unter den üblichen Bedingungen, d. h. mit Ammoniak- und Wasserstoffüberschuß bei erhöhter Temperatur und erhöhtem Druck, im allgemeinen bei 80 bis 200 °C und 80 bis 300 bar. Als Katalysatoren bei dieser Reduktion können die bekannten Verbindungen und Metalle der VIII. Nebengruppe des Periodensystems, sowie Chrom, Mangan, Kupfer, Zink, Molybdän, Wolfram, Rhenium und Cu-chromit verwendet werden, insbesondere aber Kobalt, Nickel, Eisen und Platinmetalle.

Das erfindungsgemäße Verfahren zur Herstellung primärer Amine und/oder Diamine aus Oxoverbindungen, Ammoniak und Wasserstoff in Gegenwart bekannter Hydrier-Katalysatoren, insbesondere von 3-Aminomethyl-3,5,5 trimethylcyclohexanamin (= Isophorondiamin = IPD) aus 3-Cyan-3,5,5-trimethyl-cyclohexanon (= Isophoronnitril = IPN) ist also dadurch gekennzeichnet, daß man vor der Reaktion mit Ammoniak und Wasserstoff in Gegenwart der Hydrierkatalysatoren die Oxoverbindungen bei Temperaturen von 10 bis 120 °C, vorzugsweise bei 15 bis 70 °C unter Drucken von 1 bis 300 bar einer Vorreaktion mit Ammoniak in Gegenwart von anorganischen und organischen Ionenaustauschem in der Ammoniumform als Iminbildungskatalysatoren unterwirft. Selbstverständlich kann das Verfahren als Ganzes oder in Teilschritten z. B. Vorreaktion, Wasserabscheidung, Hydrierung gestaltet werden. Es läßt sich sowohl diskontinuierlich als auch kontinuierlich durchführen. Auch kann man sowohl die Vorreaktion als auch die Hydrierung beliebig im Sumpf- oder im Rieselverfahren durchführen. Vorzugsweise wird aber die Vorreaktion im Sumpfverfahren, also mit geflutetem Katalysator, die Hydrierung dagegen im Rieselverfahren erfolgen. Hinsichtlich der Kombination der Vorreaktion und der Hydrierung kann sowohl so verfahren werden daß beide Stufen in getrennten Reaktorgefäßen durchgeführt werden, als auch in nur einem Reaktor, der gegebenenfalls in mehrere Abschnitte unterteilt ist. Zwei Reaktorgefäße sind vorteilhaft, wenn man die Verfahrensvariante in Verbindung mit der Phasentrennung durchführt.

Bei der Anwendung hoher Ammoniaküberschüsse bereits bei der Vorreaktion kann man das Verfahren auch in einem einzigen Reaktor durchführen. In diesem Fall kann der Iminbildungskatalysator entweder als separate Schicht des Kontaktbettes oder als Mischung mit dem Hydrierkontakt angeordnet sein. In dem « Gesamtreaktor » wird also sowohl die Bildung der Schiff'schen Base als auch die reduktive Aminierung in zeitlicher Aufeinanderfolge ausgeführt. Dabei können Temperatur und Druck für die Abschnitte des Reaktors unter Umständen den jeweiligen optimalen Bedingungen der Bildung der Schiff'schen Base bzw. der reduktiven Aminierung angepaßt werden.

Je nachdem, ob man die Abtrennung einer wässrigen Phase oder Ammoniaküberschuß als Maßnahme zur Begünstigung der Bildung der Schiff'schen Base bevorzugt, oder ob man zwei oder nur einen Reaktor einsetzt, ergeben sich für das erfindungsgemäße Verfahren u. a. folgende Varianten.

In der Vorreaktion wählt man ein Volumenverhältnis Oxoverbindung : $NH_3$ wie 1 : 0,5 bis 20 und unterwirft die homogene Reaktionsmischung, gegebenenfalls nach Zugabe von soviel Ammoniak, daß ein Volumenverhältnis Oxoverbindung : $NH_3$ wie 1 : 10 bis 20 entsteht, direkt der hydrierenden Aminierung. — Für die Vorreaktion wählt man ein Volumenverhältnis Oxoverbindung : $NH_3$ wie 1 : 1,3 bis 3, trennt die wässrige-ammoniakalische Phase ab und unterwirft die organische Phase nach Zugabe von soviel Ammoniak, daß ein Volumenverhältnis Oxoverbindung : $NH_3$ von 1 : 10 bis 20 entsteht, der hydrierenden Aminierung. Zur Verbesserung der Abtrennung der wässrigen von der organischen Phase kann man entweder Wasser und/oder inerte organische Lösungsmittel (nach der Bildung der Schiff'schen Base) zusetzen.

Man verwendet einen Reaktor, in dessen ersten Abschnitten der Iminbildungskatalysator und in dessen späteren Abschnitten der Hydrierkatalysator schichtenförmig angeordnet ist, und schickt eine Mischung von Oxoverbindung : $NH_3$ im Volumenverhältnis 1 : 10 bis 20 hindurch, der man mindestens von den späteren Abschnitten an — gegebenenfalls auch von Anfang an — zusätzlich Wasserstoff im erforderlichen Überschuß beifügt.

In analoger Weise kann man das Oxoverbindungs-NH₃-Gemisch unter Zusatz von Wasserstoff von späteren Abschnitten oder von Anfang an auch durch einen Reaktor shicken, der mit Mischung des Iminbildungskatalysators mit dem Hydrierungskatalysator beschickt ist.

Die jeweilige Gesamtausbeute ergibt sich aus der Ausbeute der beiden Reaktionsschritte. Beide wird man möglichst im Optimum fahren. Dabei muß das Optimum des einen Verfahrens mit dem des zweiten nicht immer übereinstimmen. Bei der Durchführung des Verfahrens wird man daher gegebenenfalls wirtschaftliche Kompromisse zwischen einzelnen Verfahrensparametern eingehen müssen, z. B. zwischen der Größe des Vorreaktors (bzw. dem Volumenverhältnis zwischen dem entsprechenden Abschnitt im Gesamtreaktor) und den übrigen Parametern (Temperatur, Verweilszeit oder IPN : NH₃-Verhältnis) (vergl. Beispiel 6). Aus der allgemeinen Literatur zur präperativen Darstellung Schiff'scher Basen bzw. zur reduktiven Aminierung ist zwar zu entnehmen, daß sich der Zusatz löslicher Salze wie Ammoniumformiat oder Ammoniumacetat und Ammoniumchlorid günstig auswirken soll, sei es dadurch, daß die Bildung Schiff'scher Basen bei sterisch gehinderten Carbonylverbindungen gefördert werden soll, oder auch durch Unterdrückung der Reduktion der Oxoverbindung zum entsprechenden Carbinol. Ihre Wirkung wird im wesentlichen auf die Einstellung bestimmter pH-Werte zurückgeführt, bei denen die Wasserabspaltung aus den zunächst sich bildenden Carbonyl-NH₃-Anlagerungsverbindungen (O-N-Ketale bzw. -Acetale-) bevorzugt erfolgt. Im allgemeinen wird auf derartige Zusätze verzichtet. Aus diesen vereinzelten, teils nur als Anmerkung bei der Darstellung spezieller Amine angeführten Hinweisen in der Literatur konnte nicht geschlossen werden, daß durch feste, im Reaktionsgemisch unlösliche Ammoniumionen tragende Ionenaustauscher eine starke katalytische Wirkung im Sinne einer heterogenen Katalyse auf die Bildung von Schiff'schen Basen ausgeübt wird. So war es überraschend, daß bei der Herstellung sonst schwierig darstellbarer Amine aus den entsprechenden Oxoverbindungen die zur Unterdrückung sonst unvermeidbarer Nebenprodukte erforderliche Vorreaktion mit NH₃ in der Dauer von mehren Stunden auf wenige Minuten herabgesetzt werden kann. Gleichzeitig konnte die Reaktionstemperatur erheblich, z. T. bis auf 10 °C, gesenkt werden. Außerdem wurde die Ausbeute an dem gewünschten Amin um einige Prozent gesteigert und die Bildung von in der Aufarbeitung störenden und die Ausbeute stark mindernden Neben-produkten fast vollständig unterdrückt.

Hinzu kommt, daß bei zusätzlicher Ausgestaltung des Verfahrens mit Ausschleusung des Reaktionswassers die Raumzeit- Ausbeute gesteigert wird. Das erfindungsgemäße Verfahren führt also nicht direkt von der Oxoverbindung zum Amin oder Diamin, sondern über die Schiff'sche Base. Mit Hilfe geeigneter Katalysatoren gelangt man zu dieser Zwischenverbindung, die wesentlich leichter der katalytischen hydrierenden Aminierung unterworfen werden kann als die Ausgangs-Oxoverbindung. Die Summe der Aktivierungsenergien dieser Teilreaktionen liegt offensichtlich niedriger als die Aktivierungs-energie für die Direktreaktion. Ohne die Gegenwart der Iminbildungskatalysatoren bildet sich bei den der Aminierung sonst schwer zugänglichen Oxoverbindungen die Schiff'sche Base nicht rasch genug, um zu einem technisch günstigen Prozeß zu gelangen. Insgesamt bewirkt die Anwendung des erfindungsgemäßen Katalysators nicht nur eine beträchtliche Ausbeutesteigerung, sondern auch eine Vereinfachung des Gesamtverfahrens durch die Verminderung des Vorreaktorvolumens und des Destillationsaufwandes bei der Reindarstellung und führt damit zu erheblichen wirtschaftlichen Einsparungen. Die nachfolgenden Beispiele dienen zur Veranschaulichung des Wesens der Erfindung.

### Beispiel 1 (Vergleichsbeispiel)

In einem mit 500 ml handelsüblichen Kobalt-Katalysator beschickten Hydrierreaktor werden 50 ml/h 3-Cyan-3,5,5-trimethylcyclohexanon (Isophoronnitril = IPN) und 500 ml/h flüssiges NH₃ von oben hineingepumpt. Das Reaktionssystem ist auf 120 °C aufgeheizt und wird mit H₂ auf 270 bar gehalten. Dabei wird ein gewisser Gasstrom eingestellt, indem über einen Abscheider 100 l/h Abgas ausgeschleust werden. Das den Reaktor unten verlassende Reaktionsgemisch gelangt über einen Kühler in den Abscheider, die Flüssige Phase wird einer Druckkolonne zugeleitet, wo das überschüssige NH₃ abdestilliert wird.

Aus dem Kolonnensumpf wird das Rohdiamin abgelassen und der weiteren Aufarbeitung zugeführt. Das so gewonnene Rohdiamin enthält praktisch kein IPN mehr, der Umsatz ist also vollständig. Die Ausbeute an 3-Aminomethyl-3,5,5-trimethylcyclohexylamin (Isophorondiamin = IPD) beträgt nach der Analyse des Gaschromatographen nur 48,3 %. Der größere Teil besteht vorwiegend aus 3-Aminomethyl-3, 5,5-trimethylcyclohexanol (Isophoronaminoalkohol = IPAA) neben 3,3,5-Trimethylcyclohexylamin (TMCA), 3,3,5-Trimethylcyclohexanol (TMC-ol), 1,3,3-Trimethyl-6-azabicyclo-[3.2.1]-octan (IPD-Imin) und einer Reihe unbekannter Verbindungen, darunter das schwer abtrennbare Peak-7-Gemisch.

### Beispiel 2 (Vergleichsbeispiel)

Es wird in einem Reaktionssystem entsprechend Beispiel 1 eine reduktive Aminierung durchgeführt, nachdem zuvor IPN mit der 10-fachen Volumenmenge NH₃ 1,5 H lang bei 70 °C vorreagiert hat. Der IPN-Umsatz ist wieder vollständig. Die IPD-Ausbeute beträgt laut Gaschromatographen 69,5 %. Der Rest besteht wiederum vorwiegend aus IPAA neben den im ersten Beispiel angeführten Vorlaufanteilen und Peak-7-Gemisch.

### Beispiel 3 (Vergleichsbeispiel)

Die reduktive Aminierung von IPN erfolgt gemäß Beispiel 2, jedoch wird die Vorreaktion 5,5 h lang bei 70° und einem Volumenverhältnis IPN : $NH_3$ von 1 : 2 und weiterer $NH_3$-Zugabe in den Reaktor zu einem Volumenverhältnis von 1 : 10 unter sonst gleichen Bedingungen durchgeführt.

Der Umsatz ist wieder vollständig. Die IPD-Ausbeute beträgt laut Gaschromatograph 90,3 %. Der Rest besteht überwiegend aus dem früher erwähnten Vorlaufanteil und nur noch zu ungefähr 2 % aus IPAA. Das schwer abzutrennende unbekannte Peak-7-Gemisch ist jadoch zu 0,6 % enthalten ; um bei der Reindarstellung von IPD diesen Anteil bis auf zulässige 0,1 % au entfernen, müssen ungefähr 5 % Verluste an IPD in Kauf genommen werden ; dadurch sinkt die Verfahrensausbeute auf etwa 85 %.

### Beispiel 4

In der gleichen Versuchsanordnung wie in Beispiel 3 wird unter Verwendung eines mit Ionenaustauscher Lewatit SP 120 gefüllten Vorreaktors und Vorreaktion von IPN mit $NH_3$ in einem Volumenverhältnis von 1 : 10 während nur 6 Minuten bei 50 °C (in einer Rohrschlange), unter sonst gleichen Bedingungen gearbeitet.

Der Umsatz ist wieder vollständig. Die IPD-ausbeute beträgt laut Gaschromatograph 94,7 %. An Nebenprodukten finden sich fast nur noch Vorlaufbestandteile, ipaa ist nur zu etwa 0,10 % vorhanden, die schwer abtrennbare Verbindung nur noch in Anteilen < 0,1 %. Nach chargenweiser Destillation der Rohprodukte verbleiben 92,0 % IPD-Reinfraktion neben 6,4 % Vorlauf und 1,6 % Nachlauf und Rückstand.

### Beispiel 5

In der gleichen Versuchsanordnung wie in Beispiel 4 wird die Vorreaktion von IPN mit $NH_3$ in einem Volumenverhältnis von 1 : 2 20 Minuten lang bei 50 °C (in einer Rohrschlange) durchgeführt. Die Hydrierung erfolgt anschließend nach weiterer Zugabe von $NH_3$ bis zu einem Volumenverhältnis von 1 : 10.

Der Umsatz ist wieder vollständig die IPD-Ausbeute beträgt laut Gaschromatographen 94,6 %. An Nebenprodukten finden sich fast nur noch Vorlaufbestandteile, IPAA ist nur zu 0,12 % vorhanden, die schwer abtrennbare Peak-7-Verbindung nur noch in Anteilen unter 0,1 %. Die chargenweise Destillation der Rohprodukte führte zu gleichen Ergebnissen wie bei Versuch 4.

### Beispiel 6

Bei den folgenden Versuchen wurden bei 4 verschiedenen Volumina des Vorreaktors das $NH_3$ : IPN-Verhältnis, bzw. die Verweilzeit und die Temperatur in den angegebenen Grenzen mehrfach im Laufe von 70 Fahrtagen variiert.

Bei den angegebenen Temperaturbereichen wurden also mindestens 2 Versuche, nämlich die bei den genannten Grenztemperaturen durchgeführt, meist jedoch auch noch weitere Versuche zusätzlich bei festen Temperaturen innerhalb des angegebenen Bereichs.

| Vorreaktor-Restvolumen ml | Molverhältnis $NH_3$ : IPN | Verweildauer [min.] | Temperaturen [°C] |
|---|---|---|---|
| 443 | 20 | 25 | 45 – 60 |
|  | 10 | 48 | 50 – 60 |
|  | 5 | 89 | 45 – 60 |
|  | 2 | 25 | 45 – 60 |
| 50 | 10 | 5,5 | 50 – 70 |
|  | 2 | 2 | 50 – 85 |
| 20 | 10 | 2,2 | 50 – 70 |
|  | 2 | 8 | 50 – 70 |
| 10 | 2 | 4 | 50 – 70 |

Bei den Versuchen mit Verweildauern von > 4 Minuten im Vorreaktor und Reaktionstemperaturen oberhalb 50 °C wurden nach der Hauptreaktion laut Gaschromatograph stets IPD-Ausbeuten um 95 % beobachtet. In allen Fällen lag dabei der IPAA-Anteil stets < 0,5%, der Anteil an schwerabtrennbarer Substanz stets < 0,15 %.

Bei den Versuchen außerhalb dieser Grenzen wurden auch geringere IPD-Ausbeuten festgestellt.

## Beispiel 7

Wie in den vorausgegangenen Beispielen wird ein Hydrierreakter (Schachtofen-Typ 1,2 l Volumen) mit 500 ml handelsüblichem Kobaltkatalysator beschickt. Durch eine Zwischenlage aus inerten Füllkörpern und ein darübergelegtes Drahtsieb abgetrennt, wird eine Füllung mit Ionenaustauscher (250 ml Schüttvolumen) aufgebaut. Das Reaktionssystem wird durch 2 getrennte Heizkreise, im unteren Bereich auf 120 °C Maximaltemperatur, im oberen dagegen auf nur 50 bis 70 °C gehalten. Von oben werden 50 ml/h IPN und 500 ml/h $NH_3$ flüssig hineingepumpt. Durch Aufdrücken von $H_2$ auf 270 bar wird das System auf Druck gehalten, wobei über einen Abscheider 200 l/h Abgas ausgeschleust werden. Das den Reaktor unten verlassende Reaktionsgemisch gelangt über einen Kühler in den Abscheider, während die flüssige Phase einer Druckkolonne zugeleitet wird, wo das überschüssige Ammoniak abdestilliert wird. Aus dem Kolonnensumpf wird das Rohdiamin abgelassen und der weiteren Aufarbeitung zugeführt. Es enthält kein IPN mehr, der Umsatz ist also vollständig. Die Ausbeute beträgt laut Gaschromatographen 94, 1 %, IPAA ist noch zu < 0,3 % enthalten, die schwer abtrennbare Substanz zu < 0,15 %.

## Beispiel 8

In einem Autoklaven mit Hubrührer werden 500 g gepulvertes IPN (= 3,03 Mol) zusammen mit 100 ml sauerem Ionenaustauscher Lewatit SP 120 eingebracht. Nach Spülen mit $N_2$ werden 125 ml flüssiges $NH_3$ (≙ 4,5 Mol) zugedrückt. Dabei steigt die Temperatur von 15 auf 22 °C, und der Rührer bleibt stehen. Nach weiterem Erwärmen läßt sich nach Erreichen von 40 °C der Rührer wieder betätigen.

Infolge spontan freiwerdender Wärme erhöht sich die Temperatur auf 65 °C. Gleichzeitig fällt der Druck von 7,2 bar auf 5,0 bar ab, der Restdruck entspricht etwa dem Überdruck an $N_2$, mit dem das flüssige Ammoniak in den Autoklaven gedrückt wurde. Nach einer Reaktionszeit von einer halben Stunde wird der Autoklav abgekühlt und entspannt, dabei entweicht nur wenig $NH_3$. Der Autoklaveninhalt bleibt auch bei Raumtemperatur flüssig. Der Ionenaustauscher wird von der flüssigen Phase abgetrennt, welche in einen Phasentrenner überführt wird, wo sie von einem kleinen Anteil wässriger Phase befreit wird. Die verbleibende ölige, organische Phase bleibt zunächst flüssig, beginnt aber bei längerem Stehen zu kristallisieren. Durch Erwärmen auf 40 °C bleibt sie jedoch flüssig. In dieser Form wird sie kontinuierlich in einem mit Kobalt beschickten Hydrierofen unter Zufügung des 10-fachen Volumens an flüssigem $NH_3$ hydriert. Dabei werden dieselben Temperatur- und Druckbedingungen wie bei den früheren Beispielen eingehalten.

Der Umsatz ist wieder vollständig ; erst bei Steigerung der Kontaktbelastung um 50 % gegenüber den früheren Beispielen beginnt laut Gaschromatographen (= GC) Aminonitril durchzuschlagen. Die IPD-Ausbeute beläuft sich auf 93,9 %, die Anteile von IPAA liegen bei A 0,5 % und die der schwer abtrennbaren Verbindung unter 0,15 %.

## Beispiel 9 (Vergleichsbeispiel)

In der gleichen Versuchsanordnung wie in Beispiel 1 wird Triacetonamin (TAA) durch aminierende Hydrierung zu Triacetondiamin (TAD) umgesetzt. Dazu wird 150 ml/h Einsatzprodukt, das zu 30,5 % Vorlauf-Anteile, 58,9 % TAA und 10,6 % höhersiedende Anteile enthält, zusammen mit 300 ml/h flüssigem $NH_3$ in den Reaktor gepumpt, der auf 130 °C aufgeheizt und mit $H_2$ auf einem Druck von 270 bar gehalten wird. Ein Gasstrom wird eingestellt, indem über den Abscheider 150 l/h Abgas ausgeschleust werden.

Das vom überschüssigem $NH_3$ befreite Reaktionsgemisch enthält laut GC-Analyse 34,9 % Vorlaufanteile, 27,6 % TAD, 9,1 % Zwischenprodukte, 27,7 % TAA-aminoalkohol und 5,7 % Höhersiedendes. Bei vollständigem TAA-Umsatz wurde somit — (bezogen auf den TAA-Gehalt des Einsatzproduktes) — eine TAD-Ausbeute von 47,0 % erzielt.

## Beispiel 10

In der gleichen Versuchsanordnung wie in Beispiel 4, also unter Verwendung eines mit Ionenaustauscher Lewatit SP 120 gefüllten Vorreaktors, wird Einsatzprodukt wie im vorigen Beispiel und flüssiges $NH_3$ in einem taa : $NH_3$-Volumenverhältnis entsprechend ca. 1 : 4 bei 70 °C während einer Verweildauer von 5 min. zur Vorreaktion gebracht und anschließend aminierend hydriert.

Das Reaktionsprodukt enthält nach Entfernung des überschüssigen $NH_3$ laut GC-Analyse nun 30,7 % Vorlauf, 56,1 % TAD, 8,2 % Zwischenprodukte, 2,3 % TAA-aminoalkohol und 2,7 % Höhersiedendes. Bei vollständigem TAA-Umsatz wurde somit — (besozen auf TAA-Gehalt des Einsatzproduktes) — eine TAD-Ausbeute von 95,2 % erzielt.

**Ansprüche**

1. Verfahren zur Herstellung primärer Amine und/oder Diamine aus Oxoverbindungen, die gegebenenfalls auch weitere zur Reduktion befähigte Gruppen enthalten können, und Ammoniak und Wasserstoff in Gegenwart bekannter Hydrier-Katalysatoren, gegebenenfalls in organischen Lösungsmitteln, insbesondere zur Herstellung von Isophorondiamin aus Isophoronnitril, dadurch gekennzeichnet, daß man vor der Reaktion mit Ammoniak und Wasserstoff in Gegenwart der Hydrierkatalysatoren die Oxo-Verbindungen bei Temperaturen von 10 bis 120 °C, vorzugsweise bei 15 bis 70 °C, und Drucken von 1 bis 300 bar, einer Vorreaktion mit Ammoniak in Gegenwart von anorganischen und organischen Ionenaustauschern in der Ammoniumform als Iminbildungskatalysatoren unterwirft.

2. Verfahren nach Anspruch 1, dadurch gekennzeichnet, daß man bei der Vorreaktion ein Volumenverhältnis von Oxo-Verbindung zu $NH_3$ wie 1 : 0,5-20 wählt und die homogene Reaktionsmischung, gegebenenfalls nach Zugabe von soviel Ammoniak, daß ein Volumenverhältnis 1 : 10-20 entsteht, direkt der hydrierenden Aminierung unterwirft.

3. Verfahren nach Anspruch 1, dadurch gekennzeichnet, daß man bei der Vorreaktion ein Molverhältnis der Oxo-Verbindung zu $NH_3$ von 1 : 3,3-3 wählt, die wäßrig-ammoniakalische Phase abtrennt und die organische Phase, gegebenenfalls nach Zugabe von soviel Ammoniak, daß ein Volumenverhältnis von 1 : 10-20 entsteht, der hydrierenden Aminierung unterwirft.

4. Verfahren nach Anspruch 3, dadurch gekennzeichnet, daß man zur Phasentrennung Wasser und/oder inerte organische Lösungsmittel zusetzt.

5. Verfahren nach den Ansprüchen 1 bis 3, dadurch gekennzeichnet, daß man nach der Vorreaktion die hydrierende Aminierung bei erhöhter Temperatur, vorzugsweise bei 80 bis 200 °C, und bei erhöhtem Druck, vorzugsweise bei 80 bis 300 bar, in Gegenwart von Hydrierkatalysatoren, vorzugsweise von solchen mit Kobalt oder Nickel als Hauptkomponenten, diskontinuierlich oder kontinuierlich durchführt.

6. Verfahren nach den Ansprüchen 1, 2 und 5, dadurch gekennzeichnet, daß man die Oxo-Verbindung und Ammoniak kontinuierlich im Volumenverhältnis wie 1 : 10-20 durch einen Reaktor schickt, in dessen ersten Abschnitten der Iminbildungskatalysator und in dessen späteren Abschnitten der Hydrierkatalysator schichtenförmig angeordnet ist, wobei durch die späteren Abschnitte des Reaktors — gegebenenfalls auch schon von Anfang an, — zusätzlich Wasserstoff hindurchgeleitet wird.

7. Verfahren nach den Ansprüchen 1, 2 und 5, dadurch gekennzeichnet, daß man die Oxo-Verbindung und Ammoniak kontinuierlich im Volumenverhältnis wie 1 : 10-20 durch einen Reaktor schickt, der gleichzeitig den Iminbildungskatalysator und den Hydrierkatalysator als Mischung enthält und man Wasserstoff nach einer Vorlaufstrecke, — gegebenenfalls auch von Anfang an, — durch den Reaktor hindurchleitet.

**Claims**

1. Process for the preparation of primary amines and/or diamines from oxo compounds which may also contain further groups capable of reduction, and ammonia and hydrogen, in the presence of known hydrogenation catalysts, if appropriate in organic solvents, in particular for the preparation of isophoronediamine from isophoronenitrile, characterised in that, before the reaction with ammonia and hydrogen in the presence of hydrogenation catalysts, the oxo compounds are subjected to a preliminary reaction with ammonia at temperatures from 10 to 120 °C, preferably at 15 to 70 °C, and under pressures of 1 to 300 bar, in the presence of inorganic and organic ion exchangers in the ammonium form as catalysts for the formation of imines.

2. Process according to Claim 1, characterised in that, in the preliminary reaction, an oxo compound/$NH_3$ volume ratio of 1 : 0.5-20 is selected and the homogeneous reaction mixture, if appropriate after the addition of such a quantity of ammonia that a volume ratio of 1 : 10-20 results, is subjected directly to the hydrogenating animation.

3. Process according to Claim 1, characterised in that an oxo compound/$NH_3$ molar ratio of 1 : 1.3-3 is selected in the preliminary reaction, the aqueous ammonia phase is separated off and the organic phase, if appropriate after the addition of such a quantity of ammonia that a volume ratio of 1 : 10-20 results, is subjected to the hydrogenating amination.

4. Process according to Claim 3, characterised in that water and/or inert organic solvents are added for phase separation.

5. Process according to Claim 1 to 3, characterised in that, after the preliminary reaction, the hydrogenating amination is carried out discontinuously or continuously at an elevated temperature, preferably at 80 to 200 °C, and under an elevated pressure, preferably under 80 to 300 bar, in the presence of hydrogenation catalysts, preferably those with cobalt or nickel as the main components.

6. Process according to Claims 1, 2 and 5, characterised in that the oxo compound and ammonia in a volume ratio of 1 : 10-20 are continuously passed through a reactor in which, in the form of Layers, the imine-formation catalyst is located in the first sections and the hydrogenation catalyst is located in the later sections, additional hydrogen being passed through the later sections of the reactor — if appropriate even from the start.

7. Process according to Claims 1, 2 and 5, characterised in that the oxo compound and ammonia in a volume ratio of 1 : 10-20 are continuously passed through a reactor which contains the imine-formation catalyst and the hydrogenation catalyst simultaneously as a mixture, and hydrogen is passed through the reactor downstream of an initial section — if appropriate even from the start.

**Revendications**

1. Procédé de fabrication d'amines et/ou de diamines primaires à partir de composés oxo, qui peuvent éventuellement aussi contenir d'autres groupes réductibles, de gaz ammoniac et d'hydrogène, en présence d'un catalyseur d'hydrogénation connu, éventuellement dans un solvant organique, en particulier pour l'obtention de l'isophorondiamine à partir d'isophoronnitrile, procédé caractérisé en ce que, avant la réaction avec l'ammoniac et l'hydrogène en présence du catalyseur d'hydrogénation, on soumet les composés oxo, à des températures de 10 à 120 °C, de préférence 15 à 70 °C, et sous des pressions de 1 à 300 bars, à une réaction préalable avec de l'ammoniac, en présence d'échangeurs d'ions minéraux ou organiques sous la forme ammonium, comme catalyseurs formateurs d'imines.

2. Procédé selon la revendication 1, caractérisé en ce que pour la réaction préliminaire, on choisit un rapport volumique du composé oxo à $NH_3$ tel que 1 : 0,5 à 20, et l'on soumet le mélange réactionnel homogène, éventuellement après addition d'une quantité d'ammoniac telle qu'il s'établisse un rapport volumique 1 : 10 à 20, directement à une amination hydrogénante.

3. Procédé selon la revendication 1, caractérisé en ce que pour la réaction préliminaire, on choisit un rapport molaire du composé oxo à $NH_3$ de 1 : 1,3 à 3, l'on sépare la phase hydro-ammoniacale, et l'on soumet la phase organique, éventuellement après addition d'une quantité d'ammoniac telle, qu'il s'instaure un rapport volumique de 1 : 10 à 20, à l'amination hydrogénante.

4. Procédé selon la revendication 3, caractérisé en ce que pour la séparation des phases, on ajoute de l'eau et/ou un solvant organique inerte.

5. Procédé selon les revendications 1 à 3, caractérisé en ce que après la réaction préalable, on effectue l'amination hydrogénante à une température élevée, de préférence 80 à 200 °C, et sous pression élevée, de préférence 80 à 300 bars, en présence de catalyseurs d'hydrogénation, de préférence ceux qui contiennent du cobalt ou du nickel comme composant principal, de manière continue ou discontinue.

6. Procédé selon les revendications 1, 2 et 5, caractérisé en ce que l'on fait passer le composé oxo et l'ammoniac, en continu, dans un rapport volumique de 1 : 10 à 20, dans un réacteur où l'on a disposé par couches, dans la première section le catalyseur formateur d'imines et dans les sections suivantes, le catalyseur d'hydrogénation, de l'hydrogène étant envoyé ensuite, en plus, dans les sections suivantes du réacteur, éventuellement même à partir du début.

7. Procédé selon les revendications 1, 2 et 5, caractérisé en ce que l'on fait passer le composé oxo et l'ammoniac en continu, dans un rapport volumique de 1 : 10 à 20, dans un réacteur qui contient en même temps le catalyseur formateur d'imine et le catalyseur d'hydrogénation, sous forme de mélange, et que l'on fait passer de l'hydrogène dans ce réacteur, après une section de départ, éventuellement même depuis le début.